# EUROPEAN PATENT SPECIFICATION

(11) **EP 0 458 432 B1**
(45) Date of publication and mention of the grant of the patent: **03.05.1995**
(21) Application number: 91301067.4
(22) Date of filing: 11.02.1991
(51) Int. Cl.: A61B 17/06

(54) **Retainer for a combined surgical suture-needle device**
Innenbeutel für eine Nähfaden und Nadel kombinierende chirurgische Vorrichtung
Pochette pour un dispositif chirurgical combinant un fil et une aiguille de suture

(30) Priority: 25.05.1990 US 529222
(43) Date of publication of application: 27.11.1991
(73) Proprietor: United States Surgical Corporation, Norwalk, Connecticut 06856 (US)
(72) Inventor: Brown, David L., Wallingford, Connecticut 06492 (US); Holzwarth, Henry A., Weston, Connecticut 06883 (US)
(74) Representative: Marsh, Roy David

(56) References cited:
- EP-A- 0 046 518
- EP-A- 0 276 964
- US-A- 3 162 307
- US-A- 4 063 638
- US-A- 4 120 395
- US-A- 4 249 656
- US-A- 4 253 563
- US-A- 4 406 363
- US-A- 4 413 727
- US-A- 4 533 041
- US-A- 4 574 948
- US-A- 4 574 957
- US-A- 4 615 435
- US-A- 4 708 241

## Description

This invention relates to a retainer for a combined surgical needle-suture device, also commonly referred to as an "armed suture" or merely a "suture", as part of a suture package. Retainers for sutures are known, inter alia, from U.S. Patent Nos. 3,857,484; 3,939,969; 3,951,261; 3,985,227; 4,063,638; 4,089,409; 4,120,395; 4,192,420; 4,249,656; 4,427,109; 4,253,563; 4,406,363; 4,412,614; 4,483,437; 4,491,218; 4,555,016; 4,572,363; 4,574,948; 4,574,957; 4,615,435; 4,708,241 and 4,813,537.

As an essential component of a suture package, the suture retainer should possess good storing qualities, provide safety in handling and permit ready access to, and removal of, the stored sutures.

The characterising features of the present invention are set out in claim 1 below, which has a prior art preamble based on the disclosure of US-A-4249656.

By way of meeting the foregoing criteria, there is provided in accordance with this invention, an armed suture retainer comprising four interconnected panels, namely, a needle retaining panel, a front cover panel connected to the needle retaining panel, a suture winding panel connected to the front cover panel and a fold-over panel connected to the suture winding panel.

The foregoing suture retainer possesses several advantages over known types of retainers, e.g., as described in U.S. Patent No. 4,249,656 referred to supra. A particularly important advantage of the suture retainer herein lies in the relative ease with which operating room personnel are able to view the needles and grasp them with forceps to effect their removal. These capabilities are made possible by the configurations of the first and second panels which, in the fully folded retainer, permit a highly visible needle display section from which each needle in the retainer can be easily removed.
In the accompanying drawings:
Fig. 1 is a plan view of one embodiment of an armed suture retainer in accordance with this invention loaded with a combined surgical needle-suture device and shown in the open condition;
Fig. 2 is a plan view of the armed suture retainer of Fig. 1 shown in the fully folded condition;
Fig. 3 is a plan view of another embodiment of an armed suture retainer in accordance with this invention shown in the fully unfolded condition;
Figs. 4, 5 and 6 are plan views of another embodiment of the armed suture retainer herein also shown in the fully unfolded, partially folded and fully folded conditions, respectively; and,
Figs. 7-10 are plan views of still other embodiments of an armed suture retainer in accordance with this invention shown in the fully unfolded condition.

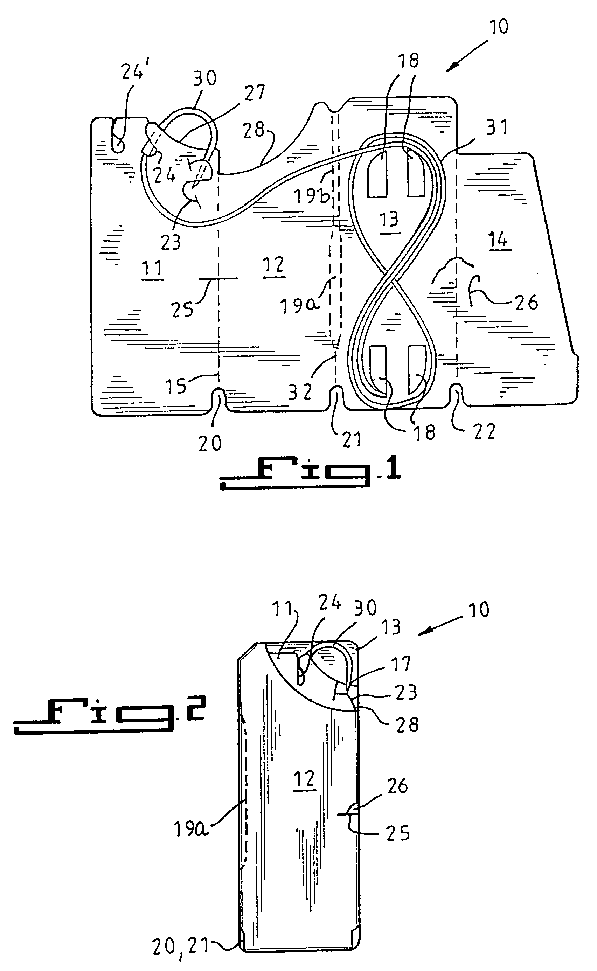

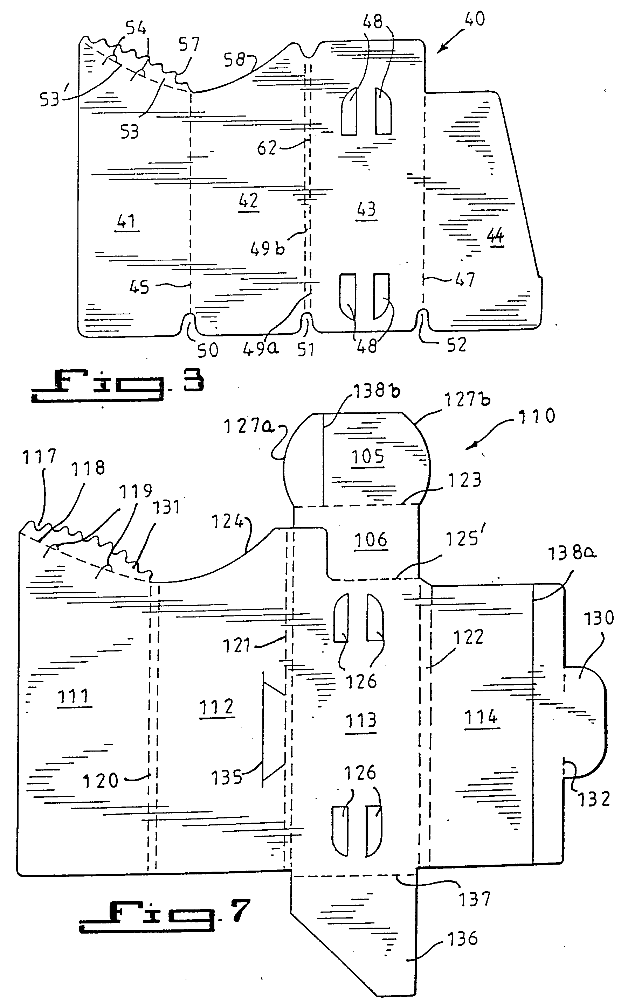

Fig. 1 illustrates a fully unfolded armed suture retainer 10 in accordance with this invention and Fig. 2 illustrates retainer 10 in the fully folded condition which it assumes when received within the pocket of an outer suture package, e.g., as described in EP-A-0498093.

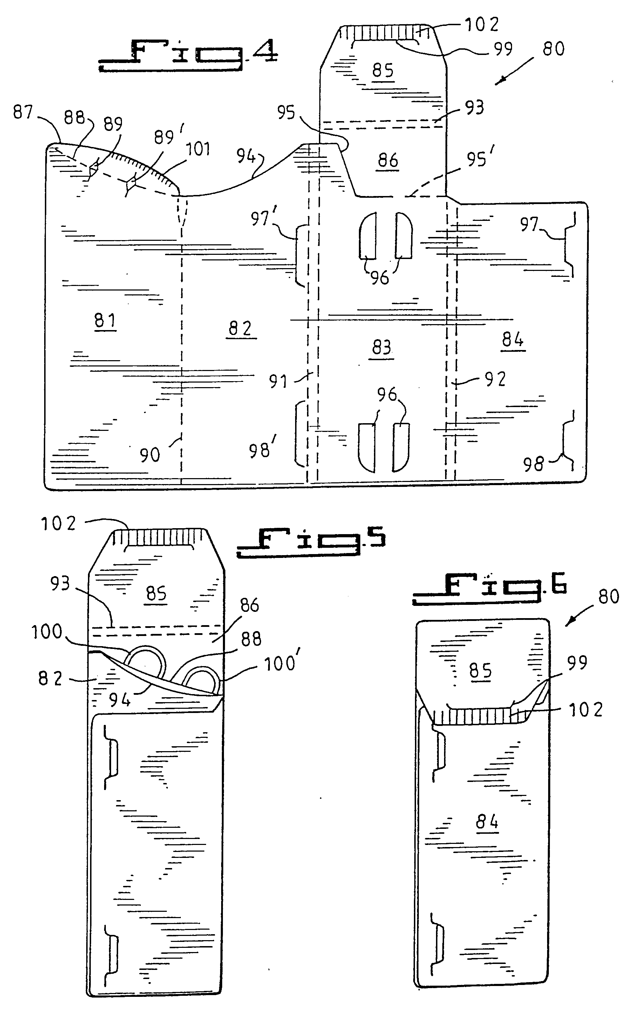

Retainer 10 is provided as a series of four interconnected panels, namely, needle retainer panel 11 possessing an upper sloping edge 27, front cover panel 12 which also possesses an upper sloping edge 28, suture winding panel 13 (which also functions as the rear panel of the fully folded retainer) and fold-over panel 14. Retainer 10 is preferably formed from a single sheet of suitable material, e.g., stiff paper or paperboard such as 5 point to 12 point solid, TYVEK (Registered Trade Mark), bleached sulfate board, plastics, foils, laminates, and the like, which is die cut to provide the desired configuration. The panels are joined to each other along perforate, or score, lines 15, 32 (and its associated gusset sections 19a and 19b) and 17. Central gusset sections 19a and 19b accommodate a limited degree of expansion of the retainer in its loaded, fully folded condition. Other gusset configurations can also be employed for this purpose, e.g., a continuous pair of parallel fold lines or perforations which provide a narrow expansion section (line 49a and 49b defining gusset 62 in the retainer of Fig. 3).

In the fully folded condition of the retainer and as shown in Fig. 2, a portion of needle 30 is readily visible in the upper section of the retainer and is easily gripped by forceps for removal. Die cut 25 cooperates with die cut 26 to provide a snap-lock feature which maintains the retainer in the fully folded condition. Rounded indentations 20, 21 and 22 serve to prevent the suture from becoming caught between the panels when folded.

To load needle component 30 with its attached suture component 31 into retainer 10, the retainer is first mounted on a winding fixture by means of loading pins (not shown) which project through openings 18 in panel 13. The point of needle 30 is then inserted in die cut 23 which is shaped somewhat like a reversed "S" by threading the point under the upper and the lower half of the reversed "S" cut. Slight tension is maintained on suture 31 from this stage of the loading procedure to its conclusion to ensure that needle 30 will maintain its placement in die cut 23 as previously described. The shank of needle 30 is then threaded through one of teardrop-shaped cutouts 24 or 24', cutout 24 being used for smaller needles (as shown in Fig. 2) and cutout 24' being used for larger needles. After panel 11 has been folded over onto panel 12, suture 31 is wound in a figure "8" pattern around the loading pins projecting through openings 18 in panel 13. Retainer 10, now loaded with needle 30 and attached suture 31, is released from the loading pins, panel 14 is folded over on panel 13 and the partly folded-over structure is given a final folding along perforate line 32 and gussets 19a and 19b. Finally, a slight counter-directional movement of the upper section of the retainer against its lower section sets the aforementioned snap-lock in place providing the fully assembled, loaded retainer of Fig. 2.

Referring to Fig. 3, there is illustrated another embodiment of the armed suture retainer of the present invention. Retainer 40 is provided as a series of four interconnected panels, namely, needle retainer panel 41 possessing an elliptical flap 53 defined by an arcuately shaped perforated fold line 53', front cover panel 42 possessing upper sloping edge 58, suture winding panel 43 which also functions as the rear panel of the fully folded retainer, and fold over panel 44. Needle retainer panel 41 possesses an arcuately serrated edge 57 of sinusoidal pattern configured to retain surgical needles thereon. The serrated edge 57 replaces cut-outs 24 and 24' and die cut 23 of the embodiment of Fig. 1 and provides an alternative means for needle retention.

Again, referring to Fig.3, a pair of inverted "J-cuts" 54 are located in spaced relation along arcuate score line 53'. When elliptical panel 53 is folded downward along the arcuate score line 53', the "J-cuts" 54 provide two parabolic openings in the elliptical panel. These openings permit sterilizing gas to readily enter the retainer in its fully folded condition. Additionally, when elliptical panel 53 is folded downward, "J-cuts" 54 provide two parabolic fingers extending upwardly and coplanar with needle retainer panel 41. These fingers provide "saddles" against or around which the needle shank and attached suture may be positioned. Panels 42 and 43 are joined together by parallel score lines 49a and 49b, to provide central gusset section 62.

Once the needles are secured within or against a sinusoidal curve of arcuately serrated edge 57 of elliptical panel 53, the latter is folded over at arcuate edge 53'. The arcuate shape of 53' prevents extension panel 53 from folding flat against retaining panel 41. When needle retaining panel 41 is folded over at perforate score 45 upon front cover panel 42, elliptical panel 53 serves to space needle retaining panel 41 from front cover panel 42, thereby greatly improving the visibility and accessibility of needle components. In this position, the needles are securely retained by engagement with serrations 54 of elliptical panel 53 and front cover panel 42.

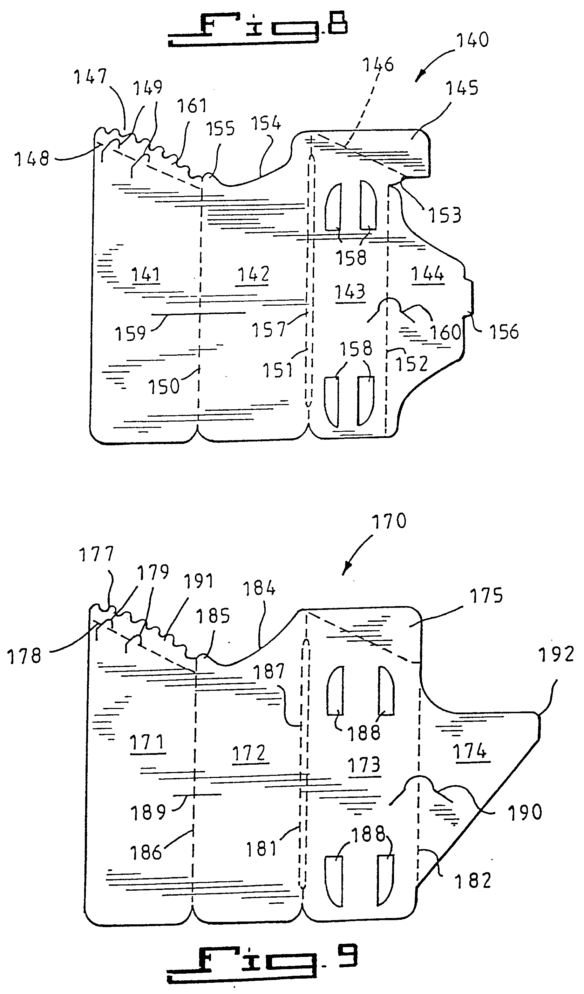

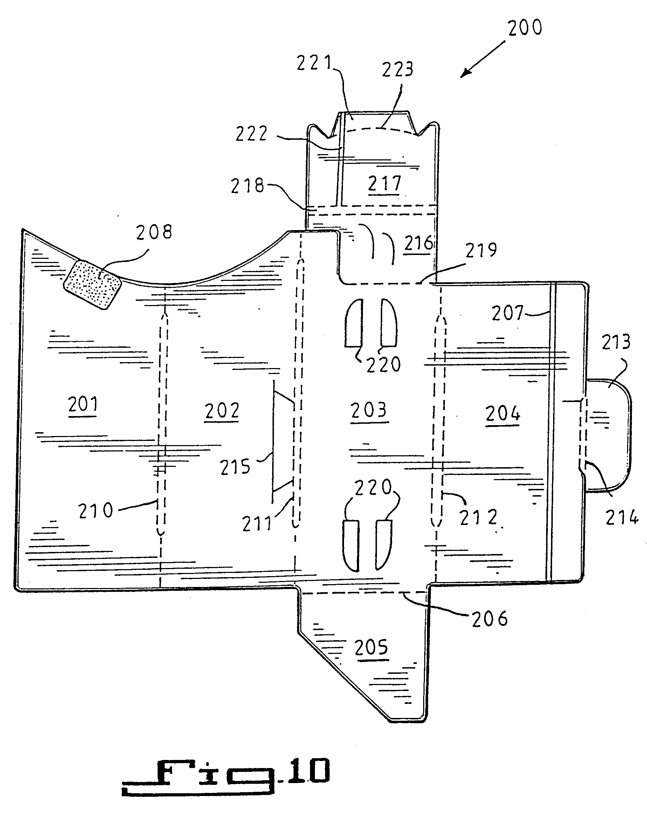

Referring to the embodiment of armed suture retainer 80 of Figs. 4-6, needle retaining panel 81 is connected to needle protecting panel 82 through single perforate score 90, panel 82 being connected to suture winding panel 83 (which is also the rear panel of the fully folded retainer) through double perforate score 91, panel 83 being connected to fold-over panel 84 through double perforate score 92. Diamond-shaped cutouts 89 and 89' are provided along upper sloping edge 88 of panel 81 in the region receiving needle components 100 and 100' (Fig. 4) of two surgical suture-needle devices. Once the needles are secured within serrations 101 of extension panel 87, the latter folds over sloping edge 88 to provide additional protection for the needles. With the needles in place and extension panel 87 folded over, needle retaining panel 81 is folded over at perforate score 90 upon needle protecting panel 82 and the surgical suture components of the combined suture-needle devices (not shown) are wound in a figure "8" pattern upon suture winding panel 83 with the aid of loading pins projecting through openings 96 in much the same manner as previously described in connection with the suture retainer embodiment of Figs. 1 and 2. Combined overlying panels 81 and 82 are thereafter folded over at double perforate score 91 upon suture winding panel 83 to slightly compress the wound sutures and retain them in place on panel 83. Panel 84 is then folded over at double perforate score 92 upon the reverse side of panel 82, die-cut locking tabs 97 and 98 on panel 84 cooperating with die-cut locking slots 97' and 98' on panel 82 to provide the nearly fully folded retainer shown in Fig. 5. Finally, outer extension panel 85 is folded over at double perforate score 93 upon both inner extension panel 86 and exposed needles 100 and 100' with die-cut tab locking 99 on panel 85 engaging the upper edge of fold-over panel 84 to provide the fully folded suture retainer shown in Fig. 6. The lower edge of inner extension panel 86 is advantageously provided with a die-cut separation line 95 and perforate score 95' which allows the entire combined extension panel 85, 86 to be conveniently separated from panel 83 or folded back upon itself thus permitting access to needles 100 and 100' from the reverse side of retainer 80 as well as from its front side. Even when combined extension 85, 86 is not-separated from panel 83, the combined extension may be folded back along separation line 94 and perforate score 95' to provide needle visibility and accessibility from the rear of retainer 80. Outer extension panel 85 possesses a knurled section 102 which facilitates the opening of fully closed retainer 80 by providing a surface for easy engagement of the lower section of extension panel 85.

Referring to Fig. 7, illustrating still another embodiment of the suture retainer of the present invention, retainer 110 features four panels, namely, needle retaining panel 111, needle protecting panel 112, suture winding panel 113 and fold-over panel 114. Double perforated scores 120 and 121 define gussets which allow for expansion of the loaded retainer in its closed condition.

Raised ribs 138a and 138b project outwards from the reverse sides of fold over panel 114 and extension panel 105, respectively. When retainer 110 is in the closed position, raised ribs 138a provide a slight concave bend to fold-over panel 114, which enables 114 to resist any tendency to thrust away from needle retainer panel 111.

Needle protection panel 112 possesses a trapezoidal shaped locking slit 135. When retainer 110 is in the fully closed position, locking tab 130 folds back at a 180° angle upon its score line 132 and cooperates with closing slit 135 of panel 112 to provide a completely secured retainer.

Along the lower edge of the suture winding panel 113 is a triangular extension panel 136 connected to winding panel 113 by single perforate score line 137. Triangular panel 136 folds over upon perforate score line 137 and secures a suture thread to retainer panel 113.

Extension panel 105 possesses convex sides 127a and 127b which facilitate the opening of retainer 110 by operating room personnel. When retainer member 110 is in a fully closed position, convex sides 127a and 127b extend outwards from the sides of retainer 80 and provide two areas for easy engagement of extension panel 105.

Needle retaining panel 111 possesses a sinusoidal serration configuration 117 and a pair of "J" cuts 119 similar in design and function to the corresponding configurations of retainer 40 of Fig. 3.

Referring to Fig. 8, there is illustrated yet another embodiment of the present invention. Retainer 140 features four panels, namely, needle retaining panel 141, front cover panel 142, suture winding panel 143, and foldover panel 144. Double perforated scores 151 define a gusset which allows for expansion of the loaded retainer in its closed condition. Front cover panel 142 possesses an upper sloping edge 154 which slopes upward to form a rounded projection 155.

Suture winding panel 143 is significantly narrower than the suture winding panels of other embodiments described herein. The narrower panel facilitates winding of the suture and minimizes the possibility that suture loops may become entangled. Suture winding panel 143 possesses triangular shaped extension panel 145 which folds over along perforated score line 146 to provide a needle protection flap in the loaded and closed condition of the retainer card. When panel 143 is folded over onto panel 142, slit 153 can be made to engage rounded projection 155 thus locking the two panels together. Bell-shaped fold-over panel 144 possesses a tab 156 which cooperates with slit 157 to secure a wound suture upon suture winding panel 143. Die cut 159 cooperates with die cut 160 to provide a snap-lock feature which maintains the retainer in the fully closed condition.

Referring to Figure 9, illustrating still another embodiment of the suture retainer of the present invention, retainer 170 possesses four panels, namely, needle-retainer panel 171, front cover panel 172, suture-winding panel 173 and fold-over panel 174. The openings 188 of suture winding panel 173 which receive the loaded pins are positioned further apart than in previous embodiments. This positioning reduces the likelihood of suture entanglement since the freedom of movement of the wound suture is limited to one direction (i.e., inwardly). In addition, loop positioning will be better achieved and secured by compression of adjoining panels more closely adjacent the fold-lines. Triangular fold-over panel 174 possesses outer edge 192 which, in the folded condition of the retainer, interlocks with slit 187 to secure the wound suture to suture winding panel 173.

Referring to Figure 10 illustrating still another embodiment of the present invention, retainer 200 possesses four panels, namely, needle retainer panel 201, front cover panel 202, suture winding panel 203, and fold over panel 204. Double perforated score lines 210, 211 and 212 constitute gussets which permit expansion of the retainer in its loaded, closed condition. Needle retainer panel 201 possesses foam block 208 which provides an alternate means of needle retention. Suture winding panel 203 possesses triangular extension panel 205 foldable along perforated line 206. Fold over panel 204 and extension panel 217 possess slightly raised ribs 207 and 222, respectively. Raised rib 207 imparts a slight concave bend to fold-over panel 204 which enables 204 to resist a tendency to thrust away from needle retainer panel 201. Raised rib 222 functions in a similar manner where extension panel 217 is concerned. Locking tab 213 is defined along double score line 214. When retainer 200 is in the fully closed condition, locking tab 213 folds back along double score line 214 to cooperate with closing slit 215 of panel 202 and provide a completely secured retainer. In the closed position, outer extension panel 217 folds over at double perforated line 218. Tab 221 folds along arcuate perforated line 223 to fit under front cover panel 202 thus providing protection for the secured needle. The retainers of the invention can be packaged within, for example, a foil package or a so-called "breather pouch" (not shown).

## Claims

1. A suture retainer (10) containing a needle suture device, the retainer comprising a first panel (11) which serves as a needle retaining panel, a second panel (12) connected to the first panel (11) and serving as a cover panel a third panel (13) connected to the second panel (12) and serving as a suture winding panel and a fourth panel (14) connected to the third panel (13) and serving as a fold over panel, the retainer being foldable to a folded condition in which the first panel (11) is folded over the second panel (12), and then the second panel (12) and first panel (11) together are folded over the third panel (13) so that the first panel (11) lies between the second (12) and third panel (13) and characterized in that the first panel (11) possesses needle securing means (23, 24) for securing the point and the shank of the needle (30) component of at least one combined surgical needle-suture device to an upper sloping edge (27) thereof, and the second panel (12) has a sloping upper edge (28), which provides exposure of each needle (30), secured to the first panel (11) in the folded condition of the suture retainer (10), above the upper sloping edges (27, 28) of said first (11) and second (12) panels.

2. A suture retainer (10) as claimed in claim 1 wherein the third panel (13) receives the suture component of at least one combined surgical needle-suture device, having for that purpose a plurality of openings (18, 96) for suture component load pins.

3. A suture retainer as claimed in claim 1 or 2 wherein the margin of each of the junctures of the first and second panels, the second and the third panels and the third and the fourth panels possesses an indentation (20, 21, 22) such that the folded condition of the retainer, the indentations prevent the suture(s) from becoming caught between the panels when the panels are folded.

4. A suture retainer as claimed in claim 1, 2 or 3 wherein the first panel possesses an extension panel (53) integral with said first panel along an upper sloping edge (27).

5. A suture retainer as claimed in claim 4 wherein said extension panel (53) is adapted to fold over upon its upper sloping edge (27) to engage a needle between said upper edge and said second panel.

6. A suture retainer as claimed in claim 4 or 5 wherein said extension panel is prevented by said edge from folding flat against said first panel.

7. A suture retainer as claimed in claim 4, 5 or 6 wherein needle securing means is so located, and the first panel (11) and the extension panel (53) so fold at the upper sloping edge (27), that the needle component(s) secured to such edge is protected.

8. A suture retainer as claimed in any one of the preceding claims wherein the means for securing a needle component thereto comprises a foam block (208).

9. A suture retainer as claimed in any one of the preceding claims wherein the means for securing a needle component to the first panel comprises a cut out associated with or defined upon said panel upper edge for accommodating passage of sterilizing gas therethrough.

10. A suture retainer as claimed in claim 4 or any one of claims 5 to 9 as dependent upon claim 4 wherein the extension panel possesses at least one serration along its free edge for further securing a needle component thereto.

11. A suture retainer as claimed in any one of the preceding claims wherein the third panel possesses an extension panel (105) which folds over upon the exposed needle(s) to provide additional protection therefor.

12. A suture retainer as claimed in claim 11 wherein the extension panel (105) is connected to the third panel by means facilitating its separation therefrom.

13. A suture retainer as claimed in claim 11 or 12 wherein the extension panel (105) is connected to the third panel by means facilitating folding of said extension panel relative to said third panel so as to provide ready needle visibility.

14. A suture retainer as claimed in any one of claims 11, 12 or 13 wherein the extension panel (105) possesses convex-shaped sides (127) such that in the folded condition of the retainer, the sides extend outwards from each side of the retainer to provide a profile which facilitates the opening of the retainer.

15. A suture retainer as claimed in any one of claims 11 to 14 wherein the extension panel possesses a locking tab for securing said extension panel to the fourth panel in the fully closed position.

16. A suture retainer as claimed in any one of claims 11 to 15 wherein the extension panel possesses a knurled gripping surface.

17. A suture retainer as claimed in any one of the preceding claims wherein the fourth panel possesses a raised rib projecting outwardly from the reverse side of the fourth panel, such that in the folded condition of the retainer, said rib imparts a concave bend to the fourth panel which counteracts any tendency of the fourth panel to thrust away from the first panel.

18. A suture retainer as claimed in claim 11 or any one of claims 12 to 17 as dependent upon claim 11 wherein the extension panel of the third panel is constructed and arranged for further securing a suture thread to said third panel.

19. A suture retainer as claimed in any one of the preceding claims wherein the second panel possesses a trapezoidal slit and the fourth panel possesses a locking tab such that in the folded condition of the retainer, said locking tab folds back at a 180° angle upon itself and cooperates with said trapezoidal slit to lock the panels of the retainer together.

20. A suture retainer as claimed in any one of the preceding claims wherein the second panel (143) possesses an upwardly rounded projection (155) and the third panel possesses a triangular-shaped extension panel (145) defined along an angled perforated line (146) and ending in a horizontal cut line (153) such that in the folded condition of the retainer said upwardly round projection (155) cooperates with said horizontal cut line to lock the panels together.

## Patentansprüche

1. Nahtmaterialaufnahme (10), enthaltend eine Vorrichtung aus Nadel und Nahtmaterial, wobei die Aufnahme umfaßt: ein erstes Feld (11), welches als Nadelhaltefeld dient, ein mit dem ersten Feld (11) verbundenes und als Deckfeld dienendes zweites Feld (12), ein mit dem zweiten Feld (12) verbundenes und als Nahtmaterialwickelfeld dienendes drittes Feld (13), sowie ein mit dem dritten Feld (13) verbundenes und als Umfaltfeld dienendes viertes Feld (14), wobei die Aufnahme in einen zusammengefalteten Zustand faltbar ist, in welchem das erste Feld (11) über das zweite Feld (12) gefaltet ist, und dann das zweite Feld (12) und das erste Feld (11) zusammen über das dritte Feld (13) gefaltet sind, so daß das erste Feld (11) zwischen dem zweiten (12) und dritten Feld (13) liegt, und dadurch gekennzeichnet, daß das erste Feld (11) Nadelbefestigungseinrichtungen (23, 24) besitzt, um die Spitze und den Schaft des Nadelbestandteils (30) von mindestens einer Vorrichtung aus kombinierter chirurgischer Nadel und Nahtmaterial an einem schrägen oberen Rand (27) desselben zu befestigen, und das zweite Feld (12) einen schrägen oberen Rand (28) aufweist, der im zusammengefalteten Zustand der Nahtmaterialaufnahme (10) für ein Freiliegen jeder am ersten Feld (11) befestigten Nadel (30) oberhalb des schrägen oberen Randes (27, 28) des besagten ersten (11) und zweiten (12) Feldes sorgt.

2. Nahtmaterialaufnahme (10) nach Anspruch 1, dadurch gekennzeichnet, daß das dritte Feld (13) den Nahtmaterialbestandteil von mindestens einer Vorrichtung aus kombinierter chirurgischer Nadel und Nahtmaterial aufnimmt, wobei es zu diesem Zweck eine Mehrzahl von Öffnungen (18, 96) für Nahtmaterialbestandteil-Bestückungsbolzen aufweist.

3. Nahtmaterialaufnahme nach Anspruch 1 oder 2, dadurch gekennzeichnet, daß der Rand von jeder der Verbindungsstellen des ersten und des zweiten Feldes, des zweiten und des dritten Feldes und des dritten und des vierten Feldes eine Einbuchtung (20, 21, 22) aufweist, so daß die Einbuchtungen im zusammengefalteten Zustand der Aufnahme verhindern, daß das Nahtmaterial zwischen den Feldern hängenbleibt, wenn die Felder zusammengefaltet sind.

4. Nahtmaterialaufnahme nach Anspruch 1, 2 oder 3, dadurch gekennzeichnet, daß das erste Feld ein Verlängerungsfeld (53) aufweist, das entlang eines schrägen oberen Randes (27) als Einheit mit dem besagten ersten Feld ausgebildet ist.

5. Nahtmaterialaufnahme nach Anspruch 4, dadurch gekennzeichnet, daß das besagte Verlängerungsfeld (53) so angepaßt ist, daß es sich auf seinem schrägen oberen Rand (27) falten läßt, um eine Nadel zwischen dem besagten oberen Rand und dem besagten zweiten Feld im Eingriff zu halten.

6. Nahtmaterialaufnahme nach Anspruch 4 oder 5, dadurch gekennzeichnet, daß das besagte Verlängerungsfeld durch den besagten Rand daran gehindert wird, sich flach gegen das besagte erste Feld anzulegen.

7. Nahtmaterialaufnahme nach Anspruch 4, 5 oder 6, dadurch gekennzeichnet, daß eine Nadelbefestigungseinrichtung so angeordnet ist, und sich das erste Feld (11) und das Verlängerungsfeld (53) so am schrägen oberen Rand (27) falten lassen, daß der (die) an einem derartigen Rand befestigte(n) Nadelbestandteil(e) geschützt ist (sind).

8. Nahtmaterialaufnahme nach einem beliebigen der vorangehenden Ansprüche, dadurch gekennzeichnet, daß die Einrichtung zum Befestigen eines Nadelbestandteils an derselben einen Schaumstoffblock (208) umfaßt.

9. Nahtmaterialaufnahme nach einem beliebigen der vorangehenden Ansprüche, dadurch gekennzeichnet, daß die Einrichtung zum Befestigen eines Nadelbestandteils am ersten Feld einen Ausschnitt umfaßt, der mit dem oberen Rand des besagten Feldes verbunden oder auf diesem gebildet ist, um Platz zu haben für einen Hindurchtritt von Sterilisiergas.

10. Nahtmaterialaufnahme nach Anspruch 4 oder einem beliebigen der von Anspruch 4 abhängigen Ansprüche 5 bis 9, dadurch gekennzeichnet, daß das Verlängerungsfeld für eine weitere Befestigung eines Nadelbestandteils an demselben mindestens einen Zahneinschnitt entlang seines freien Randes besitzt.

11. Nahtmaterialaufnahme nach einem beliebigen der vorangehenden Ansprüche, dadurch gekennzeichnet, daß das dritte Feld ein Verlängerungsfeld (105) besitzt, welches sich auf die freiliegende(n) Nadel(n) umfalten läßt, um einen zusätzlichen Schutz für diese bereitzustellen.

12. Nahtmaterialaufnahme nach Anspruch 11, dadurch gekennzeichnet, daß das Verlängerungsfeld (105) mit dem dritten Feld durch eine Einrichtung verbunden ist, die sein Abtrennen von diesem erleichtert.

13. Nahtmaterialaufnahme nach Anspruch 11 oder 12, dadurch gekennzeichnet, daß das Verlängerungsfeld (105) mit dem dritten Feld durch eine Einrichtung verbunden ist, die ein Umfalten des besagten Verlängerungsfeldes relativ zu dem besagten dritten Feld erleichtert, um so für eine rasche Sichtbarkeit der Nadeln zu sorgen.

14. Nahtmaterialaufnahme nach einem beliebigen der Ansprüche 11, 12 oder 13, dadurch gekennzeichnet, daß das Verlängerungsfeld (105) konvex geformte Seiten (127) aufweist, so daß sich die Seiten im zusammengefalteten Zustand der Aufnahme von jeder Seite der Aufnahme aus nach außen zu erstrecken, um ein Profil zu schaffen, welches das Öffnen der Aufnahme erleichtert.

15. Nahtmaterialaufnahme nach einem beliebigen der Ansprüche 11 bis 14, dadurch gekennzeichnet, daß das Verlängerungsfeld eine Verriegelungszunge aufweist, um das besagte Verlängerungsfeld im vollständig geschlossenen Zustand am vierten Feld zu befestigen.

16. Nahtmaterialaufnahme nach einem beliebigen der Ansprüche 11 bis 15, dadurch gekennzeichnet, daß das Verlängerungsfeld eine gerändelte Greifoberfläche besitzt.

17. Nahtmaterialaufnahme nach einem beliebigen der vorangehenden Ansprüche, dadurch gekennzeichnet, daß das vierte Feld eine erhöhte Rippe besitzt, die aus der Rückseite des vierten Feldes nach außen ragt, so daß die besagte Rippe im zusammengefalteten Zustand der Aufnahme dem vierten Feld eine konkave Biegung verleiht, die jeglicher Neigung des vierten Feldes zum Wegdrücken vom ersten Feld entgegenwirkt.

18. Nahtmaterialaufnahme nach Anspruch 11 oder einem beliebigen der von Anspruch 11 abhängigen Ansprüche 12 bis 17, dadurch gekennzeichnet, daß das Verlängerungsfeld des dritten Feldes für eine weitere Befestigung eines Nahtmaterialfadens an dem besagten dritten Feld geformt und angeordnet ist.

19. Nahtmaterialaufnahme nach einem beliebigen der vorangehenden Ansprüche, dadurch gekennzeichnet, daß das zweite Feld einen trapezförmigen Schlitz besitzt, und das vierte Feld eine Verriegelungszunge besitzt, so daß sich die besagte Verriegelungszunge im zusammengefalteten Zustand der Aufnahme unter einem Winkel von 180° auf sich selbst zurückfalten läßt und mit dem besagten trapezförmigen Schlitz zusammenwirkt, um die Felder der Aufnahme miteinander zu verriegeln.

20. Nahtmaterialaufnahme nach einem beliebigen der vorangehenden Ansprüche, dadurch gekennzeichnet, daß das zweite Feld (143) einen nach oben gerundeten Vorsprung (155) besitzt, und das dritte Feld ein dreieckförmiges Verlängerungsfeld (145) besitzt, das entlang einer angewinkelten perforierten Linie (146) abgegrenzt ist und in einer horizontalen Schnittlinie (153) endet, so daß der besagte nach oben runde Vorsprung (155) im zusammengefalteten Zustand der Aufnahme mit der besagten horizontalen Schnittlinie zusammenwirkt, um die Felder miteinander zu verriegeln.

## Revendications

1. Elément de retenue de suture (10) contenant un dispositif de suture à aiguille, l'élément de retenue comprenant un premier panneau (11) qui sert de panneau de retenue d'aiguille, un deuxième panneau (12) connecté au premier panneau (11) et servant de panneau couvrant, un troisième panneau (13) relié au deuxième panneau (12) et servant de panneau d'enroulement de suture et un quatrième panneau (14) relié au troisième panneau (13) et servant de panneau de repliage, l'élément de retenue pouvant être replié en un état plié dans lequel le premier panneau (11) est replié sur le deuxième panneau (12), et ensuite le deuxième panneau (12) et le premier panneau (11) ensemble sont repliés sur le troisième panneau (13) de façon que le premier panneau (11) se situe entre les deuxième (12) et troisième (13) panneaux, et caractérisé en ce que le premier panneau (11) possède un moyen d'immobilisation d'aiguille (23, 24) pour immobiliser la pointe et la tige du composant d'aiguille (30) d'au moins un dispositif chirurgical combiné de suture à aiguille sur un bord supérieur incliné (27) de celui-ci, et le deuxième panneau (12) présente un bord supérieur incliné (28) qui réalise l'exposition de chaque aiguille (30), fixée sur le premier panneau (11) dans l'état plié de l'élément de retenue de suture (10), au-dessus des bords inclinés supérieurs (27, 28) desdits premier (11) et deuxième (12) panneaux.

2. Elément de retenue de suture (10) selon la revendication 1, dans lequel le troisième panneau (13) reçoit le composant de suture d'au moins un dispositif chirurgical combiné de suture à aiguille, en ayant à cette fin une pluralité d'ouvertures (18, 96) pour des broches de charge d'un composant de suture.

3. Elément de retenue de suture selon la revendication 1 ou 2, dans lequel le bord de chacune des jonctions des premier et deuxième panneaux, des deuxième et troisième panneaux et des troisième et quatrième panneaux possède une dentelure (20, 21, 22) de sorte que dans l'état plié de l'élément de retenue, les dentelures empêchent que la ou les sutures soient prises entre les panneaux lorsque les panneaux sont repliés.

4. Elément de retenue de suture selon la revendication 1, 2 ou 3, dans lequel le premier panneau possède un panneau d'extension (53) réalisé en une pièce avec ledit premier panneau le long d'un bord supérieur incliné (27).

5. Elément de retenue de suture selon la revendication 4, dans lequel ledit panneau d'extension (53) est conçu pour être plié sur son bord supérieur incliné (27) pour la mise en prise avec une aiguille entre ledit bord supérieur et ledit deuxième panneau.

6. Elément de retenue de suture selon la revendication 4 ou 5, dans lequel ledit bord empêche ledit panneau d'extension d'être replié à plat contre ledit premier panneau.

7. Elément de retenue de suture selon la revendication 4, 5 ou 6, dans lequel le moyen de fixation d'aiguille est localisé, et le premier panneau (11) et le panneau d'extension (53) sont pliés de façon au bord supérieur incliné (27) que le ou les composants d'aiguille fixés à un tel bord soient protégés.

8. Elément de retenue de suture selon l'une des revendications précédentes, dans lequel le moyen pour fixer un composant d'aiguille sur celui-ci comprend un bloc en mousse (208).

9. Elément de retenue de suture selon l'une des revendications précédentes, dans lequel le moyen pour fixer un composant d'aiguille sur le premier panneau comprend une découpure associée à ou définie sur ledit bord supérieur de panneau pour permettre le passage d'un gaz stérilisant à travers celle-ci.

10. Elément de retenue de suture selon la revendication 4 ou l'une des revendications 5 à 9 dans la mesure où elles dépendent de la revendication 4, dans lequel le panneau d'extension possède au moins une dentelure le long de son bord libre pour fixer en outre un composant d'aiguille sur celui-ci.

11. Elément de retenue de suture selon l'une des revendications précédentes, dans lequel le troisième panneau possède un panneau d'extension (105) qui est replié sur la ou les aiguilles exposées pour constituer une protection additionnelle pour celles-ci.

12. Elément de retenue de suture selon la revendication 11, dans lequel le panneau d'extension (105) est relié au troisième panneau par un moyen facilitant sa séparation de celui-ci.

13. Elément de retenue de suture selon la revendication 11 ou 12, dans lequel le panneau d'extension (105) est relié au troisième panneau par un moyen facilitant le pliage dudit panneau d'extension relativement audit troisième panneau de façon à réaliser une visibilité aisée de l'aiguille.

14. Elément de retenue de suture selon l'une des revendications 11, 12 ou 13, dans lequel le panneau d'extension (105) possède des côtés de forme convexe (127) de façon que dans l'état plié de l'élément de retenue, les côtés s'étendent vers l'extérieur depuis chaque côté de l'élément de retenue afin de réaliser un profil qui facilite l'ouverture de l'élément de retenue.

15. Elément de retenue de suture selon l'une des revendications 11 à 14, dans lequel le panneau d'extension possède une patte de verrouillage pour fixer ledit panneau d'extension au quatrième panneau dans l'état entièrement fermé.

16. Elément de retenue de suture selon l'une des revendications 11 à 15, dans lequel le panneau d'extension possède une surface de saisie molettée.

17. Elément de retenue de suture selon l'une des revendications précédentes, dans lequel le quatrième panneau possède une nervure relevée faisant saillie vers l'extérieur depuis le côté arrière du quatrième panneau de telle sorte que dans l'état plié de l'élément de retenue, ladite nervure donne une courbure concave au quatrième panneau qui agit contre toute tendance du quatrième panneau d'être poussé au loin du premier panneau.

18. Elément de retenue de suture selon la revendication 111 ou l'une des revendications 12 à 17 dans la mesure où elles dépendent de la revendication 11, dans lequel le panneau d'extension du troisième panneau est construit et agencé pour immobiliser encore plus un fil de suture sur ledit troisième panneau.

19. Elément de retenue de suture selon l'une des revendications précédentes, dans lequel le deuxième panneau possède une fente trapézoïdale, et le quatrième panneau possède une patte de verrouillage de sorte que dans l'état plié de l'élément de retenue, ladite patte de verrouillage est repliée suivant un angle de 180° sur elle-même et coopère avec ladite fente trapézoïdale pour verrouiller ensemble les panneaux de l'élément de retenue.

20. Elément de retenue de suture selon l'une des revendications précédentes, dans lequel le deuxième panneau (143) possède une saillie arrondie vers le haut (155), et le troisième panneau possède un panneau d'extension de forme triangulaire (145) défini le long d'une ligne perforée angulaire (146) et se terminant par une ligne découpée horizontale (153) de sorte que dans l'état plié de l'élément de retenue, ladite saillie arrondie vers le haut (155) coopère avec ladite ligne découpée horizontale pour verrouiller les panneaux ensemble.
